(19) Europäisches Patentamt
European Patent Office
Office européen des brevets

(11) EP 0 901 491 B1

(12) # FASCICULE DE BREVET EUROPEEN

(45) Date de publication et mention de la délivrance du brevet:
**22.01.2003 Bulletin 2003/04**

(21) Numéro de dépôt: **97924064.5**

(22) Date de dépôt: **07.05.1997**

(51) Int Cl.[7]: **C07D 451/02**, A61K 31/46

(86) Numéro de dépôt international:
**PCT/FR97/00825**

(87) Numéro de publication internationale:
**WO 97/043285 (20.11.1997 Gazette 1997/50)**

(54) **DERIVES DU TROPANE UTILISABLES EN PARTICULIER POUR LA DETECTION IN VIVO DES TRANSPORTEURS DE LA DOPAMINE**

TROPANDERIVATE INSBESONDERE VERWENDBAR ZUR DETEKTION VON DOPAMIN TRANSPORTERS

TROPANE DERIVATIVES USEABLE IN PARTICULAR FOR IN VIVO DETECTION OF DOPAMINE TRANSPORTERS

(84) Etats contractants désignés:
**AT BE CH DE DK ES FI FR GB GR IE LI LU NL PT SE**

(30) Priorité: **10.05.1996 FR 9605839**

(43) Date de publication de la demande:
**17.03.1999 Bulletin 1999/11**

(73) Titulaire: **SCHERING AKTIENGESELLSCHAFT**
**13342 Berlin (DE)**

(72) Inventeurs:
- **MAUCLAIRE, Laurent**
  **F-75013 Paris (FR)**
- **EDMOND, Patrick**
  **F-37210 Parcay-Meslay (FR)**
- **GUILLOTEAU, Denis**
  **F-37260 Fondettes (FR)**
- **BESNARD, Jean-Claude**
  **F-37210 Rochecorbon (FR)**
- **FRANGIN, Yves**
  **F-37340 Hommes (FR)**

(74) Mandataire: **Audier, Philippe André et al**
**Brevalex,**
**3, rue du Docteur Lancereaux**
**75008 Paris (FR)**

(56) Documents cités:
**WO-A-95/11901** **WO-A-96/39198**
**US-A- 5 310 912**

- **JOURNAL OF MEDICINAL CHEMISTRY, vol. 37, no. 10, - 1994 WASHINGTON US, pages 1535-1542, XP002023291 MARK M. GOODMAN ET AL: "Synthesis and characterization of radioiodinated ..."**
- **CHEMICAL ABSTRACTS, vol. 123, no. 13, 25 Septembre 1995 Columbus, Ohio, US; abstract no. 164212e, KUNG, MEI-PING ET AL: "IPT: a novel iodinated ligand for the CNS dopamine transporter" XP002023292 & SYNAPSE(N.Y.), vol. 20, no. 4, - 1995 pages 316-324,**

Remarques:
Le dossier contient des informations techniques présentées postérieurement au dépôt de la demande et ne figurant pas dans le présent fascicule.

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

### Domaine technique

La présente invention a pour objet des dérivés du tropane, qui peuvent être radioiodés et utilisés comme radioligands iodés pour la visualisation en SPECT(Single Photon Emission Computed Tomography) des transporteurs de la dopamine au niveau du système nerveux central.

La dopamine est un neuromédiateur qui est synthétisé au niveau du neurone présynaptique où il est stocké. Lcrsque le neurone est stimulé, la dopamine est libérée dans la fente synaptique où elle diffuse. Une partie inceragit avec les récepteurs du neurone postsynaptique. Cette interaction provoque des réactions biochimiques intracellulaires qui conduisent entre autre à la propagation du signai nerveux. La plus grande partie est recaptée par le neurone présynaptique grâce à un transporteur. L'inhibition du transport de la dopamine, par les dérivés de la cocaïne en particulier, conduit à une augmentation du taux de dopamine au niveau post synaptique du neurone. Des anomalies de la neurotransmission dopaminergique sont impliquées dans des maladies neurodégérératives ou psychiatriques telles que les maladies de Parkinson et d'Alzheimer, et la schizophrénie. Etant donné le rôle important du transporteur de la dopamine dans la régulation de la neurotransmission, le développement de radioligands émetteurs de rayonnement gamma susceptibles de se fixer sur le transporteur de la dopamine avec une forte affinité et sélectivité est nécessaire pour la visualisation de ce transporteur, dans le but de faire le diagnostic précoce de ces maladies et dans le but de pouvoir apprécier :

- l'évolution de la densité de transporteurs de la dopamine au cours d'une maladie, et
- le suivi d'une thérapie administrée à un patient.

### Etat de la technique antérieure

Dans ce but, plusieurs types de ligands ont été étudiés, tels que les dérivés du GBR de formule :

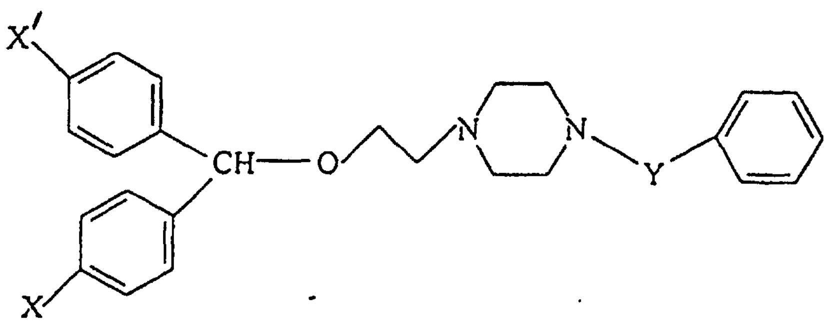

A titre d'exemple de tels dérivés, on peut citer les composés suivants répondant à la formule ci-dessus :

GBR 12935 : X=X'=H ; Y=-$(CH_2)_3$- Kd = 5,5 nM (Andersen 1987)
GBR 12783 : X=X' =H ; Y = -$CH_2$-CH=CH- Kd = 1,6 nM (Bonnet et Conscancin 1986)

Ce type de composés possède in vitro une très bonne affinité pour le transporteur de dopamine. Néanmoins, des études de compétition in vitro avec un analogue iodé du GBR 12783 (Foulon 1992) ont mis en évidence une perte importante de l'affinité pour le transporteur de la dopamine. De plus, des études de biodistribution cérébrale chez le rat ont révélé une fixation non spécifique très importante.

Plus récemment, on a proposé l'utilisation d'analogues iodés de la cocaïne qui possèdent tous la structure de base du tropane, représentée ci-dessous :

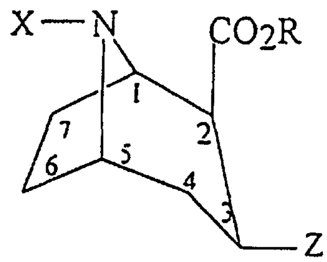

dans laquelle X, R et Z peuvent être divers substituants.

Des composés de ce type sont décrits dans les documents WO-A-92/02260(1) , WO-A-93/09814(2), WO-A-93/18033(3), WO-A-94/04146(4), WO-A-95/11901(5), J. of Med. Chem., 1992, Vol 35, n°6, pages 969-981(6), J. Med. Chem., 1994, 37, pages 1535-1542(7) ; J. Med. Chem., 1995, 38, pages 379-388(3) et J. Med. Chem., 1996, 39, pages 543-548(9).

Parmi, ces dérivés du tropane, on peut distinguer une première famille de composés dans iesquels X de la formule ci-dessus représente $CH_3$ et Z représente un groupe phényle substitué ou non, et une deuxième famille de composés dans lesquels le substituant X représente le groupe propényle éventuellement substitué par un atome d'iode, et Z représente un groupe phényle substitué.

Parmi ces dérivés, ceux de la première famille dans lesquels Z représente un groupe phényle substitué par un atome d'iode, ont plus particulièrement été développés, par exemple le composé RTI 55 ou β-CIT illustré dans WO-A-92/02260(1). Cependant, ce composé a l'inconvénient de ne pas présenter une sélectivité suffisante pour les transporteurs de la dopamine car il se fixe également sur les transporteurs de la sérotonine. Aussi, pour une visualisation du système transporteur de la dopamine, il est nécessaire d'attendre suffisamment longtemps pour que la fixation non spécifique de ce dérivé sur les transporteurs de la sérotonine soit éliminée.

Les dérivés de la deuxième famille tels que l'iodoaltropane (X = iodopropényle, Z = parafluorophényle) décrit dans WO-A-95/11901(5) et dans J. Nucl. Med., 1996, 37, pages 1197-1202(10), et le [$^{125}$I] IPT (X = iodopropényle, Z = p-chlorophényle) décrit dans le document J. Med. Chem. 1994, 37, pages 1535-1542(7) et Synapse, 1995, 20, pages 316-324(11), présentent une affinité et une spécificité pour les transporteurs de la dopamine. Toutefois, il serait intéressant d'améliorer cette affinité, cette spécificité ainsi que les propriétés cinétiques de composés de ce type, pour disposer de radio-ligands plus efficaces, permettant d'obtenir une visualisation des systèmes transporteurs de la dopamine plus rapidement.

**Exposé de l'invention**

La présente invention a précisément pour objet de nouveaux dérivés du tropane qui présentent de meilleurs propriétés que les composés connus, en ce qui concerne la cinétique de fixation dans le cerveau, l'affinité pour les transporteurs de la dopamine et la spécificité pour ces transporteurs par rapport à ceux de la sérotonine. De plus, ces nouveaux dérivés peuvent être utilisés pour le diagnostic de maladies telles que la maladie de Parkinson.

Aussi, l'invention a pour objet un composé de formule :

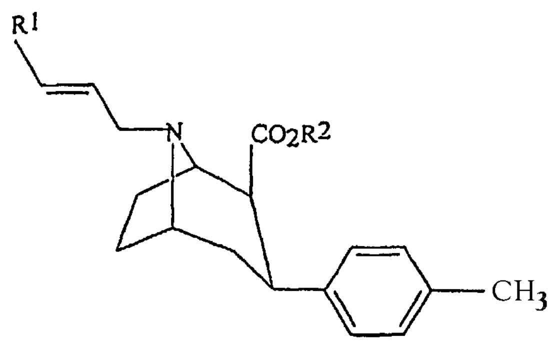

(I)

dans laquelle :

- $R^1$ représente I, un isotope radioactif de I ou un groupe de formule $Sn(R^3)_3$ dans laquelle $R^3$ est un groupe alkyle de 1 à 8 atomes de carbone ; et
- $R^2$ représente H ; un groupe alkyle en $C_1$ à $C_6$ ; un groupe phényle ; un groupe phényle substitué par un atome d'halogène, un groupe méthyle ou un groupe méthoxy ; un groupe phénylalkyle ou phénylalkényle dont le groupe alkyle ou alkényle comprend 1 à 6 atomes de carbone et dont le groupe phényle est éventuellement substitué par un atome d'halogène ;w un groupe cycloalkyle en $C_3$ à $C_8$.

Ce composé est donc un dérivé du tropane dans lequel le marqueur (isotope radioactif de l'iode) se trouve fixé par l'intermédiaire d'un groupe propényle sur l'atome d'azote de la structure de base du tropane, le tropane étant substitué par ailleurs par un groupe phényle substitué par un groupe méthyle.

De préférence, dans la formule (I) donnée ci-dessus $R^2$ représente un groupe alkyle, en particulier le groupe méthyle.

Lorsque $R^1$ représente un atome d'iode radioactif, celui-ci peut être $^{125}I$, $^{123}I$ ou $^{131}I$ car ces isotopes conviennent pour une visualisation in vivo des transporteurs de la dopamine en SPECT (Single Photon Emission Computed Tomography), en raison de leur période radioactive relativement courte.

Pour cet examen, les composés de l'invention peuvent être utilisés sous la forme d'isomères purs ou de mélange d'isomères. Toutefois, on obtient de meilleurs résultats lorsque l'on utilise un composé avec $R^1$ en position E.

$R^1$ peut aussi représenter le groupe $Sn(R^3)$ avec $R^3$ étant un groupe alkyle ayant de 1 à 8 atomes de carbone, par exemple le groupe n-butyle. De tels dérivés sont des produits intermédiaires intéressants pour la préparation de dérivés iodés et radioiodés.

Les composés de l'invention peuvent être préparés par des procédés classiques tels que celui donné par Goodman et al dans J. Med. Chem., 1994, 37, pages 1535-1542. Cette synthèse correspond au schéma réactionnel illustré sur la figure 1.

En raison de leur spécificité et de leur affinité pour les transporteurs de la dopamine, les composés de l'invention peuvent être utilisés dans des compositions pharmaceutiques et radiopharmaceutiques. De telles compositions comprennent le composé de formule (I) décrit ci-dessus dans laquelle $R^1$ représente I ou un isotope radioactif de I et $R^2$ a la signification donnée ci-dessus, et un véhicule pharmaceutiquement acceptable. Dans le cas des compositions radiopharmaceutiques, $R^1$ représente un isotope radioactif de I.

Ces compositions peuvent être en particulier sous la forme de solutions injectables comprenant de $10^{-5}$ à $10^{-3}$ μg/l du composé. Pour réaliser la visualisation in vivo des transporteurs de la dopamine dans le système nerveux central, on peut injecter au patient à examiner une quantité appropriée de solution, puis on examine le patient dans un scanner SPECT 0,5 à 2 heures après l'injection. La dose injectée peut être de 0,005 à 0,5 μg par kg de poids corporel, soit une radioactivité de 35 MBq à 370 MBq par injection.

D'autres caractéristiques et avantages de l'invention apparaîtront mieux à la lecture des exemples suivants en référence aux dessins annexés.

## Brève description des dessins

La figure 1 illustre un schéma réactionnel de synthèse des composés de l'invention.

La figure 2 est un diagramme illustrant la fixation d'un dérivé de l'invention sur le cervelet, le striatum et le cortex frontal, exprimée en pourcentage de la dose injectée par gramme de tissu.

La figure 3 est un diagramme illustrant la fixation d'un composé de l'art antérieur (β-CIT) sur le cervelet, le striatum et le cortex frontal dans les mêmes conditions que la figure 2, celle-ci étant exprimée également en pourcentage de la dose injectée par gramme de tissu.

La figure 4 illustre les résultats obtenus pour la visualisation des transporteurs de la dopamine chez le primate en fonction du temps.

## Exposé détaillé des modes de réalisation

Sur la figure 1, on a représenté les schémas de synthèse possibles des composés de l'invention.

Dans ce schéma de synthèse, on part d'un ester de l'anhydroecgonine **1** que l'on fait réagir avec le bromure de (méthyl phényl) magnesium. On obtient ainsi le composé **2** que l'on transforme en composé **3** par réaction avec du trichloroéthylchloroformate et traitement avec un mélange d'acide acétique et de zinc en copeaux.

On transforme ensuite le composé **3** en composé **5** soit directement par réaction avec du 3-chloro-1-trialkylstannyl-prop-1-éne, soit en deux étapes par réaction avec du 1-bromo-2-propyne pour obtenir le composé **4** que l'on transforme en composé **5** avec l'hydrure de trialkylstannyl. Le composé **5** peut être transformé en composé **6** non radioactif par réaction avec de l'iode en présence de chloroforme, ou en composé radioactif par réaction avec de l'iodure de sodium Na $^{125}I$ en présence d'un agent oxydant comme l'eau oxygénée.

**Exemple 1 : Préparation du 2-β-carbométhoxy-3β-paraméthylphényle)-8- (3- [125I] iodoprop-2E-ényl) nortropane (7)**

Ce composé correspond au composé **7** de la figure 1 avec $R^2 = CH_3$.

Pour cette préparation, on suit le mode opératoire de la figure 1.

**a) Préparation du 2β-carbométhoxy-3β(p-méthylphényl)tropane (2)**

L'anhydroecgonine méthyl ester **1** (leq.) est dissous dans 25 ml d'éther anhydre puis ajouté à 5 eq. de bromure de(méthylphényl) magnésium à -40°C. Après 3 heures d'agitation mécanique, le mélange est refroidi à -78°C et traité goutte à goutte par une solution de 5 ml d'acide trifluoroacétique dilué dans 25 ml d'éther anhydre. Après une heure d'agitation, le mélange est ramené à 0°C, puis traité par 70 ml d'eau et 26 ml d'HCl concentré. La phase aqueuse est ensuite traitée par $NH_4OH$ et extraite à l'éther (3 x 40 ml). Les différentes phases organiques sont rassemblées, lavées à la saumure, séchées sur $Na_2SO_4$ et évaporées pour donner le composé **2** avec un rendement de 30 %.

Les caractéristiques RMN (200 MHz, $CDCl_3$) sont données dans le tableau 1.

**b) Préparation du 2β-carboinéthoacy-3β (p-méthyl phényl)nortropane (3)**

Un mélange du composé **2** obtenu précédemment (1 eq.) et de trichloroéthylchloroformate (5,5 eq) est porté à 120°C et agité pendant 75 min. Après retour à la température ambiante, le trichloroéthylchloroformate en excès est distillé sous pression réduite. Le carbamate brut ainsi obtenu est dissous dans 15 ml (1 eq.) d'acide acétique, puis traité par du zinc en copeaux (25 eq.). Le mélange est agité à la température ambiante pendant 16 heures, traité par de la célite et filtré. Le filtrat est extrait au chloroforme, lavé à la saumure, séché sur $Na_2SO_4$ et évaporé. Cette réaction conduit après purification par chromatographie flash (silice, $Et_2O$, $Et_3N$ 75/25) au composé **3** avec un rendement de 70 %.

**c) Préparation du 2β-carbométhoxy-3β(p-méthylphényl)-8-(3-tributylstannylprop-2E-ènyl)nortropane (5)**

Le composé **3** obtenu précédemment (1 eq.) et le 3-chloro-1E-(tri-n-butylstannyl)prop-1-ène (1 eq) sont dissous dans 2 ml/eq d'éthanol absolu contenant une quantité catalytique de KI. Le mélange est agité et chauffé à 70°C pendant 16 heures. Après retour à la température ambiante, le mélange est traité par $NaHCO_3$ et extrait à l'éther. La phase organique est lavée à la saumure, séchée sur $Na_2SO_4$ et évaporée pour donner le composé **5**. Chaque produit est purifié sur plaque préparative de silice en utilisant comme phase éluante éther de pétrole/AcOEt 9/1.

Caractérisation RMN (200 MHz, $CDCl_3$) :

Pour ce produit, on observe :

- Un doublet à 5,96 ppm; J3 = 19 Hz, i = 1H
- un doublet de doublet dédoublé à 5,80ppm, i = 1H

**d) Préparation du 2β-carbométhoxy-3β-(p-méthylphényl)-8-(3- [125I] iodoprop-2E-ènyl) nortropane (7)**

A une solution contenant 50 µg du composé **5** obtenu en c), 50 µl de HCl 0,1 N et 50 µl de $H_2O_2$ 3 % p/v, on ajoute 5 µl de $Na^{125}I$ (37 MBq/ml, 1mCi/ml). La réaction est laissée à la température ambiante pendant 15 minutes, puis arrêtée par l'addition de 100 µl d'une solution de bisulfite de sodium (300 mg/ml). Le milieu réactionnel est ensuite rendu alcalin par une solution de $NaHCO_3$ (500 mg/ml) et extrait avec AcOEt (3 x 1 ml). La phase organique est évaporée par un courant d'azote et le produit brut est purifié par chromatographie liquide à haute performance (HPLC) selon les conditions opératoires suivantes :

Colonne C18; débit 1ml/min; phase mobile MeOH/$H_2O$/$Et_3N$(75/25/0,2) Temps d'élution = 13 min.

La fraction contenant le produit désiré est collectée, concentrée sur Sep Pak C18, puis extraite par 2x1ml de $CHCl_3$. Le chloroforme est évaporé par un courant d'azote. Le résidu est repris par une solution aqueuse.

**Exemple 2 : Préparation du 2β-carbométhoxy-3β-(p-méthylphényl)-8(3 iodoprop-2E-ényl)nortropane (6)**

Dans cet exemple, on suit le même mode opératoire que dans l'exemple 1, pour préparer le composé **5** de l'étape c) de l'exemple 1.

Ce composé **5** est traité par une solution de $I_2$ (1M) dans du $CHCl_3$ à 0°C, jusqu'à l'apparition d'une ,couleur jaune. Le mélange est ensuite lavé par $NaHCO_3$ à 10 % dans l'eau, séché sur $Na_2SO_4$ et évaporé. Le produit brut est

purifié sur plaque préparative de silice en utilisant comme phase éluante éther de pétrole/AcOEt/9/1.
Caractérisation RMN (200 MHz, $CDCl_3$) :
Pour ce produit, on observe :

- Un doublet à 6,23 ppm ; J3 = 14,3, i = 1H; un massif à 6,50 ppm, i = 1H.

Les caractéristiques RMN (200 MHz, $CDCl_3$) des composés intermédiaires obtenus dans l'exemple 1 et les rendements des réactions sont donnés dans le tableau 1.
Pour les produits intermédiaires obtenus dans l'étape c), on observe :

- un doublet à 5,96 ppm ;
- J3 = 19Hz
- i = 1H ; un doublet de doublet dédoublé à 5,80 ppm, i = 1 H.

Dans le tableau 2, on a mentionné le composé de l'exemple 1 et deux composés de l'art antérieur : l'IPT décrit dans les documents 7 et 11, et l'iodoaltropane décrit dans les documents 5 et 10.

**Exemple 3**

Dans cet exemple, on teste in vivo chez le rat l'affinité et la spécificité du composé de l'exemple 1 dénommé ci-après PE21, pour le transporteur de la dopamine.
A cet effet, on utilise 3 lots de rats mâles de la souche Wistar en utilisant 6 rats par lot. Le lot numéro 1 reçoit tout d'abord une injection intraveineuse de GBR 12909 (inhibiteur spécifique du transporteur de la dopamine, fabriqué par RBI Bioblock) à une dose de 5 mg/kg, puis on lui injecte 30 minutes après par voie intraveineuse le composé de l'exemple 1, à une dose de $8.10^{-6}$ mg/kg.
Le lot numéro 2 reçoit tout d'abord une injection de paroxétine (inhibiteur spécifique du transporteur de la sérotonine, fabriqué par les laboratoires Beecham) à une dose de 5 mg/kg, puis 30 minutes après, une injection du composé de l'exemple 1, dans les mêmes conditions que le lot numéro 1.
Le lot numéro 3 ou lot témoin reçoit uniquement une injection intraveineuse du composé de l'exemple 1.
2 heures plus tard, on sacrifie les animaux et on détermine les doses de radioactivité présentes dans les tissus du cervelet, du striatum et du cortex frontal.
Les résultats obtenus sont donnés sur la figure 2 et ils sont exprimés en % de la dose injectée par gramme de tissu.
Sur cette figure, les colonnes en blanc se rapportent au lot numéro 3 (témoin), les colonnes striées se rapportent au lot numéro 1 ayant reçu GBR 12909, et les colonne en pointillé se rapportent au lot numéro 2 ayant reçu la paroxétine.
Sur cette figure, on remarque que la fixation du composé de l'exemple 1 conforme à l'invention dans le striatum est prévenue à 74 % par une préinjection de GBR 12909. Le composé de l'invention est donc spécifique du transporteur de la dopamine puisqu'une saturation de ce transporteur par un inhibiteur spécifique (GBR 12909) empêche sa fixation.

**Exemple comparatif 1**

Dans cet exemple, on suit le même mode opératoire que dans l'exemple 3 pour effectuer le même essai de biodistribution cérébrale in vivo chez le rat mais en utilisant le composé de l'art antérieur β-CIT (3β-4-iodo[$^{125}$I]phényl)-tropan-2β-carboxylic acid methyl ester) au lieu du composé de l'exemple 1.
Les résultats obtenus sont représentés sur la figure 3, en utilisant les mêmes symboles.
Sur cette figure, on remarque que la fixation du composé de l'art antérieur dans le striatum n'est inhibée que de 31 %. Cette fixation résiduelle de [$^{125}$I]-βCIT est donc due à une liaison à des sites non transporteurs de la dopamine, qui est beaucoup plus importante que celle obtenue avec le composé de l'invention
Par ailleurs, dans le cortex frontal riche en sites transporteurs de la sérotonine, la fixation du composé de l'art antérieur [$^{125}$I]-βCIT est plus importante que celle du composé de l'invention. De plus, elle est inhibée à 55 % par une préinjection de paroxétine.
Ainsi, on voit que le composé de l'invention possède une très forte spécifité pour le transporteur de la dopamine par rapport à celui de sérotonine, ce qui n'est pas le cas avec β-CIT qui se lie à ces deux transporteurs.
Par ailleurs, le composé de l'invention possède une très forte spécificité pour le transporteur de la dopamine dans le striatum à des temps précoces (2 heures).
En effet, la fixation non spécifique dans le striatum déterminée après saturation par le GBR 12909 est de 25 % environ alors qu'elle est d'environ 70 % pour le β-CIT

**Exemple 4**

Dans cet exemple, on effectue également une étude de biodistribution chez le rat du composé de l'exemple 1 en suivant le même mode opératoire que dans l'exemple 3 mais en sacrifiant les animaux une 1/2 heure, 1 heure ou 4 heures après l'injection.
Les résultats obtenus sont donnés dans le tableau 3. Dans ce tableau, on a reporté également les résultats obtenus dans l'exemple 3.
Ces résultats montrent que le produit s'accumule de façon préférentielle dans le striatum et que le rapport maximum striatum sur cortex frontal ou cervelet est obtenu pour des temps précoces (entre 0,5 et 1 h après injection).

**Exemple 5**

Dans cet exemple, on effectue des études in vitro sur des préparations membranaires cérébrales.
Ces études sont des études de compétition entre [$^3$H]-GBR 12935 (transporteur de la dopamine), [$^3$H]-paroxétine (transporteur de la sérotonine), [$^3$H]-nisoxétine (transporteur de la noradrénaline), et le dérivé iodé de l' exemple 1 (PE21).
Les constantes d'inhibition Ki (nM) obtenues avec le composé de l'invention PE21 en compétition avec les différents ligands sont données dans le tableau 4.
A titre comparatif, on a reporté également dans le tableau 4, les résultats obtenus avec le ligand β-CIT, soit le 2β-carbométhoxy-3β-(4'-iodophényl)-tropane, et les résultats donnés dans le tableau 1 du document 10 : J. Nucl. Med., 1996, 37, pages 1197-1202 sur des essais du même type effectués avec le β-CIT et l'iodoaltropane décrit dans WO-A-95/11901.
Au vu de ce tableau, on constate que le composé de l'invention PE21 présente une meilleue spécificité (Ki (5-HT) : Ki (DA)=29,4) pour les transporteurs de la dopamine que l'iodoaltropane (Ki(5-HT) : Ki (DA)=25).
De même, si l'on compare l'affinité de ces deux composés à celle du β-CIT, on remarque que le rapport des Ki,DA (iodoaltropane)/DA (β-CIT) est égal à 6 alors que le rapport des Ki,DA (PE21)/DA(β-CIT) est égal à 0,63, ce qui démontre la meilleure affinité (10 fois plus d'affinité) du composé PE21 de l'invention.
De même, le composé PE21 de l'invention présente une meilleure spécificité et une meilleure affinité que le composé IPT de l'art antérieur (document 11).
En effet, dans le document 11, on peut relever que la constante d'affinité de l'IPT pour les transporteurs de dopamine est Kd=0,25±0,02 nM (tableau 1) et que cette constante d'affinité pour les transporteurs de sérotonine est Kd=1,2±0,02 nM (page 320). On obtient ainsi une spécificité Kd(5-HT)/Kd(DA) d'environ 5.
Avec le composé PE21 de l'invention, cette spécificité est nettement supérieure car Ki(5-HT)/Ki(DA) est égal à 29,4, comme on peut le voir dans le tableau 4.
Ainsi, PE21 est 5 fois plus spécifique que IPT.
On a aussi déterminé in vitro l'affinité du composé [$^{125}$I]PE21 pour les transporteurs de la dopamine, et on a obtenu comme valeur :

$$Kd=0,09\pm0,01 \text{ nM.}$$

A titre compararatif, l'affinité du composé IPT pour les transporteurs de la dopamine donnée dans le document 11 correspond à :

$$Kd=0,25\pm0,02 \text{ nM.}$$

Il ressort donc de ces résultats que PE21 a une affinité 2,5 fois plus élevée que le composé proche IPT.
Ces résultats mettent en évidence la supériorité du composé PE21 de l'invention par rapport aux composés les plus proches de l'art antérieur. Or, il n'était pas prévisible que le remplacement d'un atome de Cl ou F par un groupe méthyle puisse conduire à une telle supériorité biologique.

**Exemple 6**

Dans cet exemple, on effectue une étude cinétique in vivo de la distribution cérébrale du composé de l'exemple 1, chez le primate (macaque Cynomologus, femelle de 2 kg, anesthésiée à la kétamine, en effectuant une acquisition de données toutes les 12 minutes au CERASPECT).
Les résultats de cette étude sont illustrés sur la figure 4.

Sur cette figure, on voit que le composé de l'exemple 1 se lie de manière spécifique au niveau des noyaux gris centraux, et qu'une image de ces structures est obtenue entre 1 heure et 2 heures après l'injection.
En revanche, le composé de l'art antérieur β-CIT ne permet d'obtenir une image spécifique de ces structures qu'à partir de 20 heures après l'injection.
Le composé de l'invention est donc très intéressant pour la visualisation in vivo du système transporteur de la dopamine.

**Exemple 7**

Dans cet exemple, on a testé le composé de l'exemple 1 PE21 chez le rat dans un modèle de maladie de Parkinson obtenu avec une lésion unilatérale du passage nigrostriatal. Dans ce but, on a infusé des rats de souche Wistar unilatéralement dans la substantia nigra avec 8 μg de 6-OHDA(6-hydroxy dopamine).
Trois semaines plus tard, on leur a administré une injection intraveineuse de 3,7 MBq de $[^{125}I]$PE21.
Deux heures plus tard, on a sacrifié les rats et récupéré leurs cerveaux. Ceux-ci ont été gelés et on a découpé des sections coronales de 20 μm d'épaisseur que l'on a exposé sur des hyperfilms β-max pendant six semaines.
Les rapports striatum/cortex frontal ont été calculés après analyse des films. Sur le côté de la lésion, on a observé un rapport moyen de 0,86 ± 0,10 alors que ce rapport était de 7,20 ± 0,91 sur le côté intact.
Ces résultats obtenus sur des autoradiogrammes ex vivo démontrent que PE21 convient pour détecter in vivo des lésions spécifiques des cellules dopaminergiques.

TABLEAU 1

| Caractérisation RMN (200 Mhz, $CDCl_3$) | | | | |
|---|---|---|---|---|
| Composé | Ph-$CH_3$ | N-$CH_3$ | $CO_2CH_3$ | H aromatiques |
| Ex 1<br>étape a)<br>rendement : 30 % | s,i = 3, 2, 11 | s,i=3,2,17 | s,i=3,3,23 | 2d, 1=4,<br>6,93-7,06 |
| Ex 1<br>étape b)<br>rendement : 70 % | s,i=3,2,33 | | s,i = 3, 3, 40 | S,i = 4, 7, 11 |

TABLEAU 2

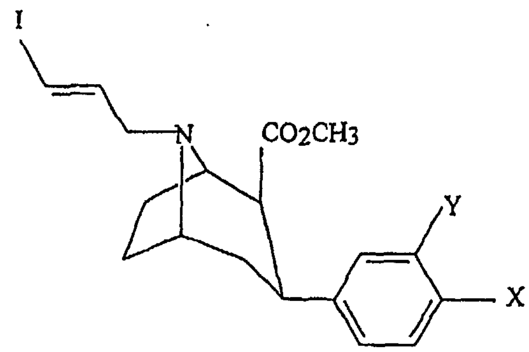

| Composé | X | Y |
|---|---|---|
| Exemple 1 | $CH_3$ | H |
| IPT | Cl | H |
| Altropane | F | H |

TABLEAU 3

| Temps post-injection (heures) | Cervelet | Striatum (% dose injectée/g tissu) | Cortex frontal (% dose injectée/g tissu) |
|---|---|---|---|
| 0,5 | 0,100 ± 0,015 | 1,061 ± 0,132 | 0,124 ± 0,020 |
| 1 | 0,055 ± 0,010 | 0,537 ± 0,102 | 0,070 ± 0,016 |
| 2 | 0,038 ± 0,010 | 0,272 ± 0,084 | 0,039 ± 0,06 |
| 4 | 0,021 ± 0,002 | 0,074 ± 0,030 | 0,018 ± 0,004 |

TABLEAU 4

| Ligand | Affinité : Ki (nM) | | | Sélectivité |
|---|---|---|---|---|
| | Dopamine (DA) | Sérotomine 5-HT | Noradrénaline | D5-HT/DA |
| PE21 (composé ex 1) | [$^3$H]GBR 17 ± 7 | [$^3$H]paroxétine 500 ± 80 | [$^3$H]nisoxétine > 100 | 29,4 |
| β-CIT | [$^3$H]GBR 27 ±2 | [$^3$H]paroxétine 30 ± 2 | [$^3$H]nisoxétine 80 ± 28 | 1,11 |

TABLEAU 4 (suite)

| Ligand | Affinité : Ki (nM) | | | Sélectivité |
|---|---|---|---|---|
| | Dopamine (DA) | Sérotomine 5-HT | Noradrénaline | D5-HT/DA |
| Iodoaltropane (document 10) | [$^3$H]Win35428 6,62 ± 0,78 | [$^3$H]citalopram 182 ± 41,8 | | 25 |
| β-CIT (document 10) | [$^3$H]WIN35428 1,08 ± 0,6 | [$^3$H]citalopram 2,53 ± 0.02 | | |

**Références citées**

1 : WO-A-92/02260
2 : WO-A:93/09814
3 : WO-A-93/18033
4 : WO-A-94/04146
5 : WO-A-95/11901
6 : J. Med. Chem., 1992, vol 35, n°6, pages 969-981.
7 : J. Med. Chem., 1994, vol 37, pages 1535-1542.
8 : J. Med. Chem., 1995, vol 38, pages 379-388.
9 : J. Med. Chem., 1996, vol 39, pages 543-548.
10 : J. Nucl. Med., 1996, vol 37, pages 1197-1202.
11 : Synapse, 1995, 20, pages 316-324.


**Revendications**

**1.** Composé de formule :

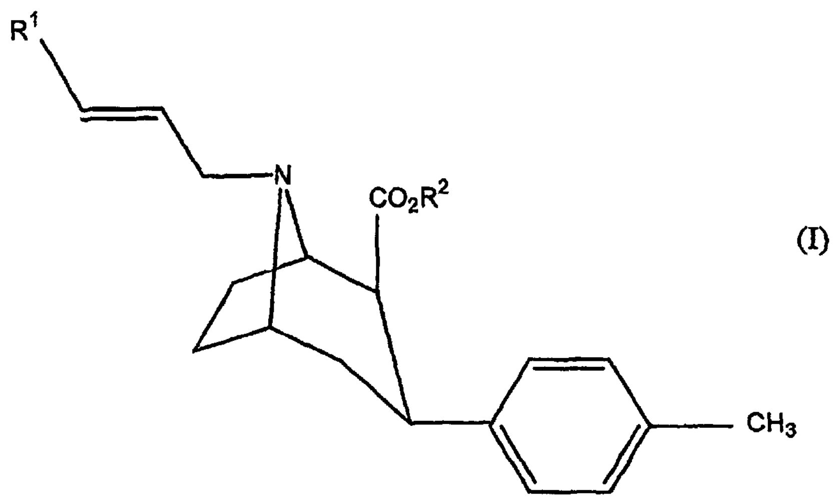

(I)

dans laquelle :

- $R^1$ représente I, un isotope radioactif de I ou un groupe de formule $Sn(R^3)_3$ dans laquelle $R^3$ est un groupe alkyle de 1 à 8 atomes de carbone ; et
- $R^2$ représente H ; un groupe alkyle en $C_1$ à $C_6$ ; un groupe phényle ; un groupe phényle substitué par un atome d'halogène, un groupe méthyle ou un groupe méthoxy ; un groupe phénylalkyle ou phénylalkényle dont le groupe alkyle ou alkényle comprend 1 à 6 atomes de carbone et dont le groupe phényle est éventuellement substitué par un atome d'halogène ; ou un groupe cycloalkyle en $C_3$ à $C_8$.

**2.** Composé selon la revendication 1, **caractérisé en ce que** $R^2$ est le groupe méthyle.

**3.** Composé selon la revendication 1 ou 2, **caractérisé en ce que** $R^1$ représente $^{125}I$, $^{123}I$ ou $^{131}I$.

**4.** Composé selon la revendication 1, **caractérisé en ce que** $R^1$ représente $Sn(C_4H_9)_3$ , $^{123}I$, $^{125}I$ ou $^{131}I$ et $R^2$ représente $CH_3$.

**5.** Composé selon l'une quelconque des revendications 1 à 4, **caractérisé en ce que** $R^1$ se trouve en position E.

**6.** Procédé de préparation d'un composé répondant à la formule :

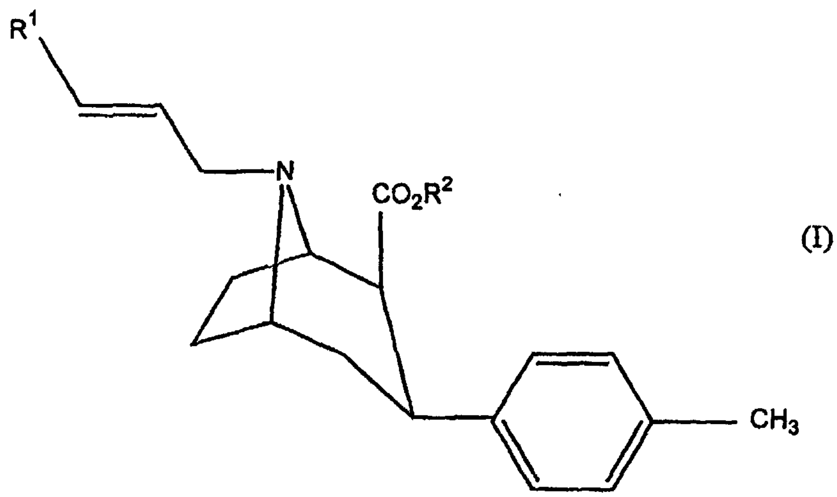

(I)

dans laquelle $R^1$ représente I et $R^2$ est tel que défini dans la revendication 1, **caractérisé en ce qu'**il comprend les étapes suivantes :

1) réaction du bromure d'arylmagnésium de formule :

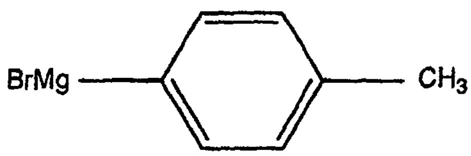

avec un ester de l'anhydroecgonine <u>1</u> de formule :

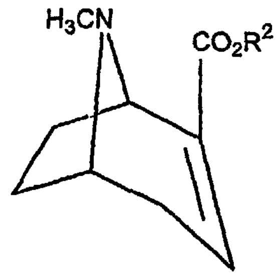

1

dans laquelle $R^2$ a la signification donnée ci-dessus pour obtenir un composé <u>**2**</u> de formule :

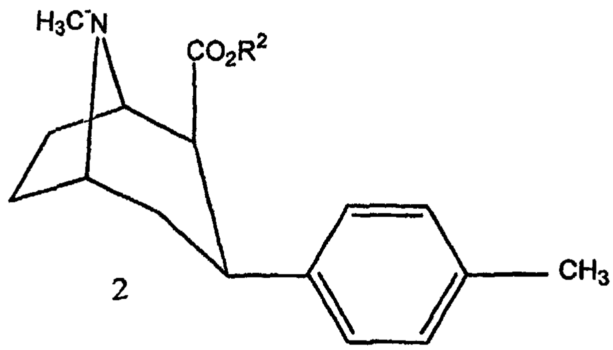

2

2) transformation du composé **2** en composé **3** de formule :

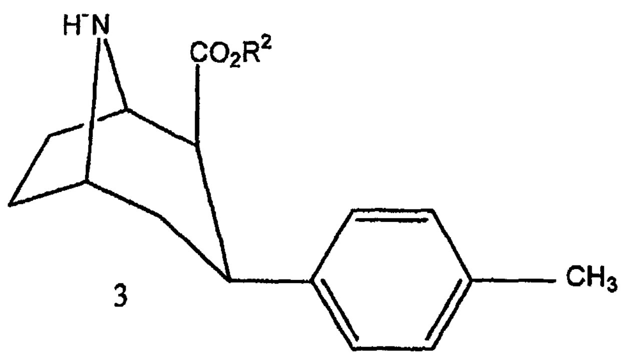

3

par réaction avec du trichloroéthyl chloroformate et réduction avec du zinc et de l'acide acétique,
3) conversion du composé **3** en composé **5** de formule :

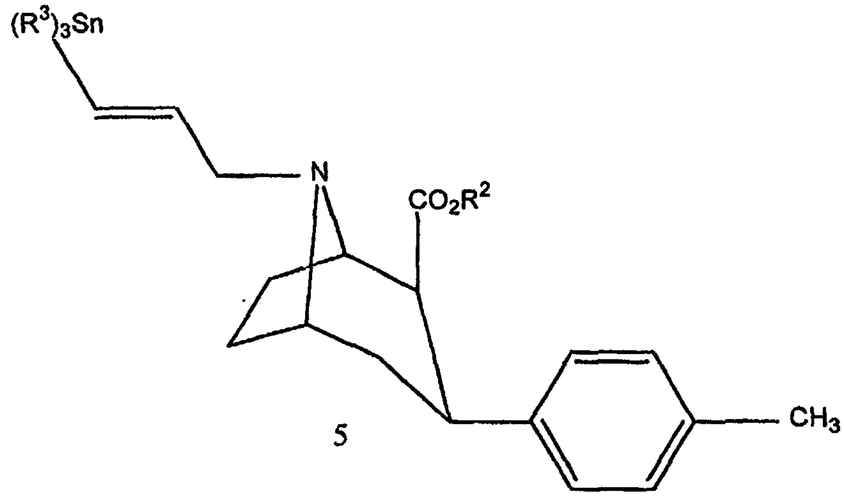

5

dans laquelle $R^3$ est un groupe alkyle, et

4) réaction du composé 5 avec de l'iode pour obtenir le composé de formule (I) défini ci-dessus.

**7.** Procédé de préparation d'un composé répondant à la formule :

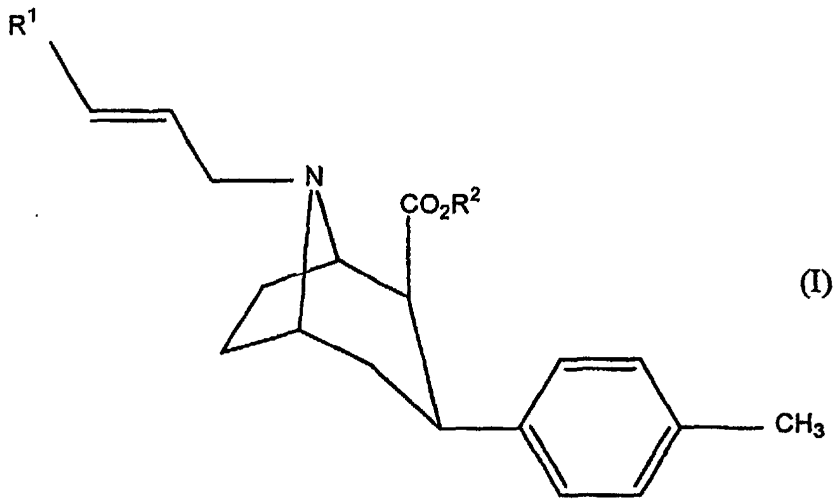

(I)

dans laquelle $R^1$ représente un isotope radioactif de I et $R^2$ est tel que défini dans la revendication 1, **caractérisé en ce qu'**il comprend les étapes suivantes :

1) réaction du bromure d'arylmagnésium de formule :

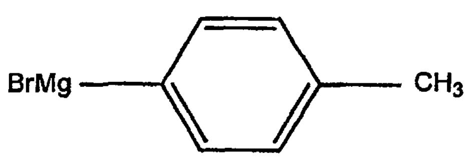

avec un ester de l'anhydroecgonine 1 de formule :

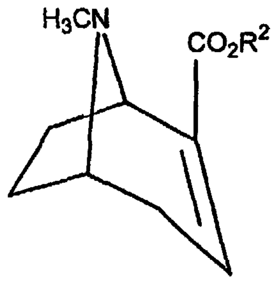

1

dans laquelle $R^2$ a la signification donnée ci-dessus pour obtenir un composé **2** de formule :

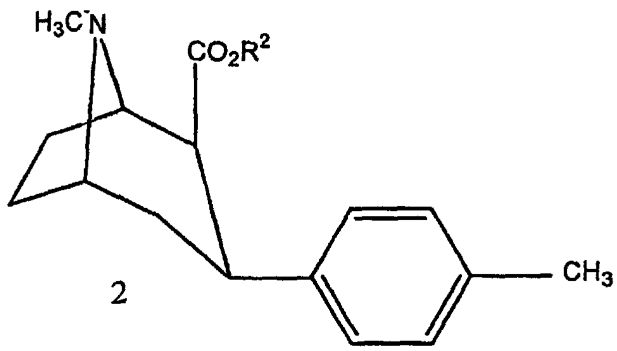

2

2) transformation du composé **2** en composé **3** de formule :

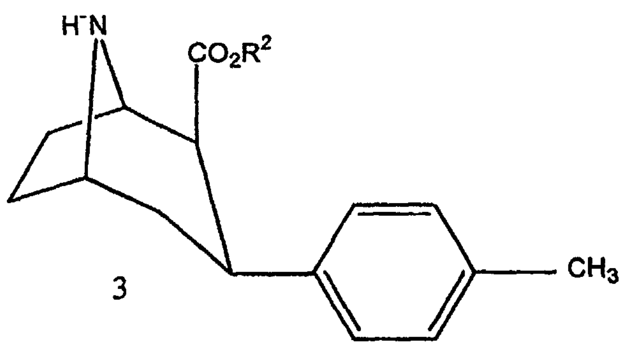

3

par réaction avec du trichloroéthyl chloroformate et réduction avec du zinc et de l'acide acétique,
3) conversion du composé **3** en composé **5** de formule :

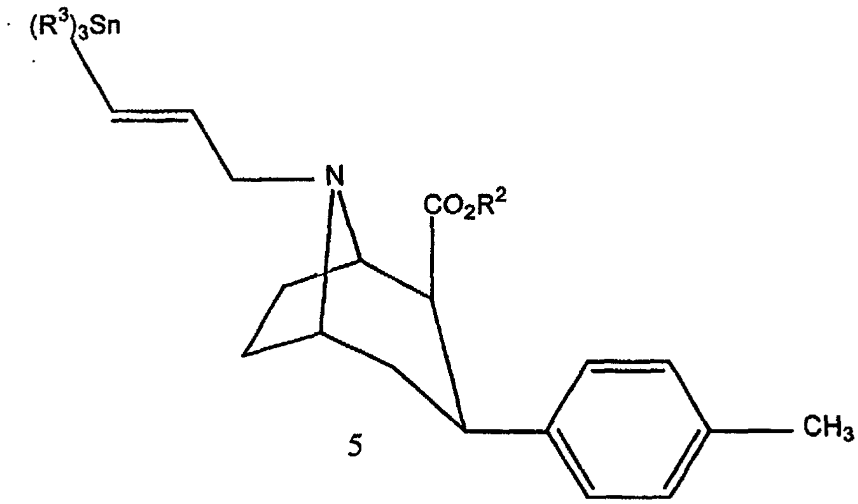

5

dans laquelle $R^3$ est un groupe alkyle, et

4) réaction du composé 5 avec l'iodure de formule Na*I dans laquelle *I est un isotope radioactif de l'iode, en présence d'un agent oxydant.

**8.** Procédé selon l'une quelconque des revendications 6 et 7, **caractérisé en ce que** dans l'étape 3) on convertit directement le composé 3 en composé 5 par réaction avec du 3-chloro-1-trialkylstannyl-prop-1-ène.

**9.** Procédé selon l'une quelconque des revendications 6 et 7, **caractérisé en ce que** dans l'étape 3) on fait réagir tout d'abord le composé 3 avec du 1-bromo-2-propyne pour obtenir le composé 4 de formule :

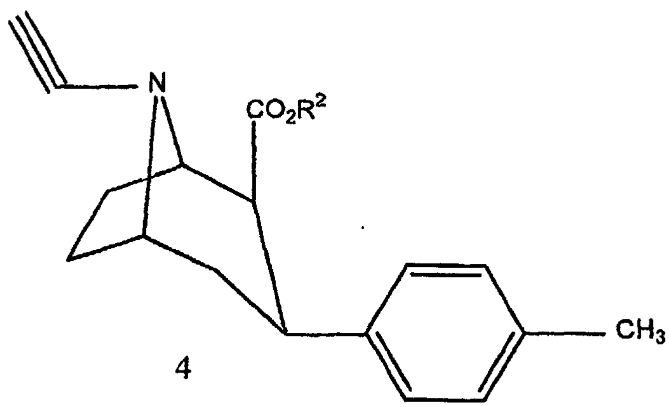

4

puis, on fait réagir le composé 4 avec de l'hydrure de trialkylstannyl pour obtenir le composé 5.

**10.** Composition pharmaceutique, **caractérisée en ce qu'**elle comprend un composé de formule :

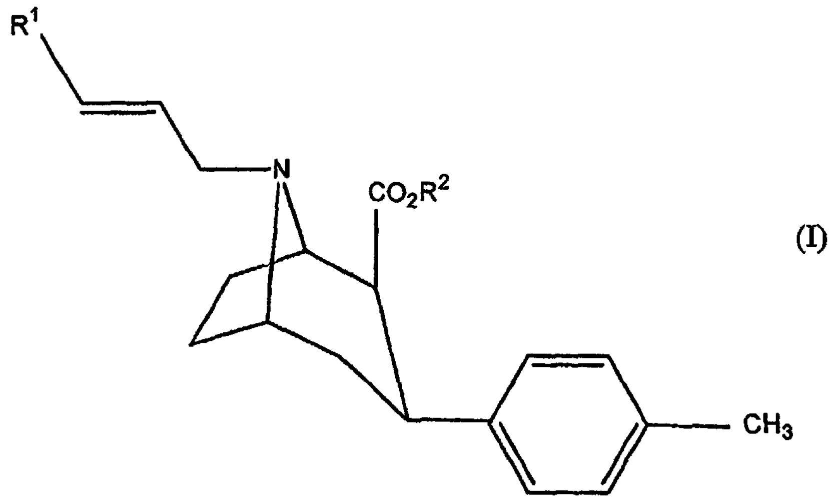

(I)

dans laquelle $R^1$ représente I ou un isotope radioactif de I ; et $R^2$ est tel que défini dans la revendication 1, et un véhicule pharmaceutiquement acceptable.

**11.** Composition radiopharmaceutique, **caractérisée en ce qu'**elle comprend un composé de formule :

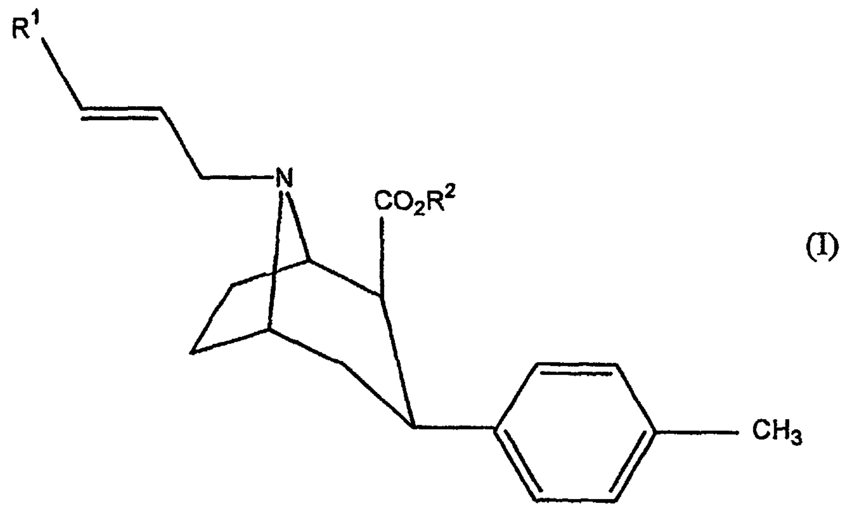

(I)

dans laquelle $R^1$ représente un isotope radioactif de I ; et $R^2$ est tel que défini dans la revendication 1, et un véhicule pharmaceutiquement acceptable.

**12.** Composition selon l'une quelconque des revendications 10 et 11, **caractérisée en ce qu'**elle est sous forme de solution injectable.

**13.** Composition selon l'une quelconque des revendications 10 à 12, **caractérisée en ce que** $R^1$ représente $^{125}I$, $^{123}I$ ou $^{131}I$, $R^2$ représente $CH_3$, et $R^1$ est en position E.

**14.** Composition radiopharmaceutique selon l'une quelconque des revendications 11 à 13, pour la visualisation des transporteurs de la dopamine dans le système nerveux central.

**Patentansprüche**

1. Verbindung mit der Formel:

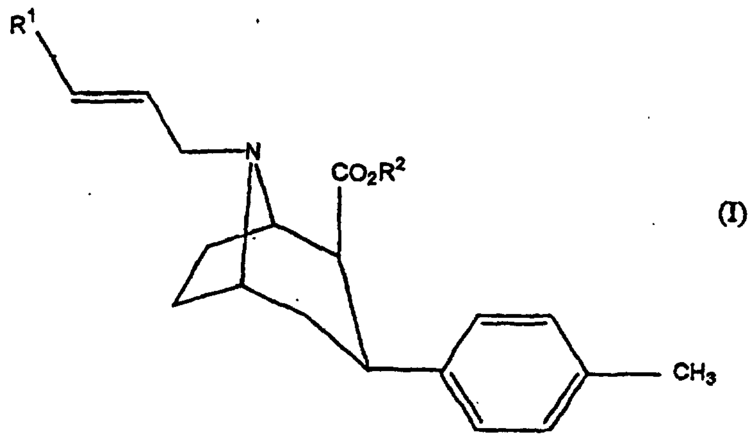

(I)

in der:

- $R^1$ I, ein radioaktives Isotop von I oder eine Gruppe mit der Formel $Sn(R^3)_3$ darstellt, in der $R^3$ eine Alkylgruppe mit 1 bis 8 Kohlenstoffatomen ist; und
- $R^2$ H; eine $C_1$-$C_6$-Alkylgruppe; eine Phenylgruppe; eine Phenylgruppe, die durch ein Halogenatom substituiert ist; eine Methyloder eine Methoxygruppe; eine Phenylalkyloder Phenylalkenylgruppe, wobei die Alkyloder Alkenylgruppe 1 bis 6 Kohlenstoffatome umfasst und die Phenylgruppe gegebenenfalls durch ein Halogenatom substituiert ist; oder eine $C_3$-$C_8$-Cycloalkylgruppe darstellt.

2. Verbindung nach Anspruch 1, **dadurch gekennzeichnet, dass** $R^2$ die Methylgruppe ist.

3. Verbindung nach Anspruch 1 oder 2, **dadurch gekennzeichnet, dass** $R^1$ $^{125}I$, $^{123}I$ oder $^{131}I$ darstellt.

4. Verbindung nach Anspruch 1, **dadurch gekennzeichnet, dass** $R^1$ $Sn(C_4H_9)_3$, $^{123}I$, $^{125}I$ oder $^{131}I$ und $R^2$ $CH_3$ darstellt.

5. Verbindung nach einem der Ansprüche 1 bis 4, **dadurch gekennzeichnet, dass** $R^1$ sich in der E-Stellung befindet.

6. Verfahren zur Herstellung einer Verbindung, die der folgenden Formel entspricht:

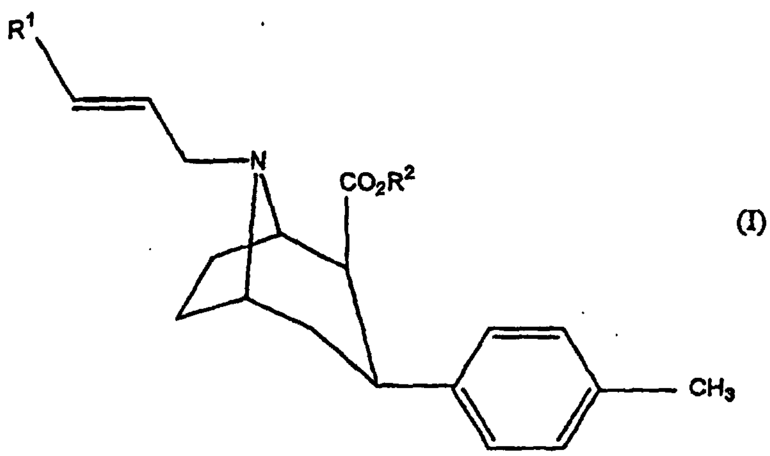

(I)

in der $R^1$ I darstellt und $R^2$ wie in Anspruch 1 definiert ist, **dadurch gekennzeichnet, dass** es die folgenden Stufen

umfasst:

1) Umsetzung des Arylmagnesiumbromids der Formel:

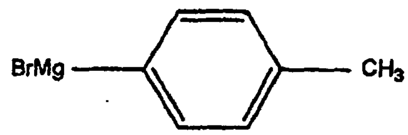

mit einem Ester des Anhydroecgonins 1 der Formel:

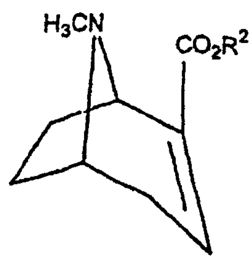

1

in dem $R^2$ die nachstehend gegebene Bedeutung hat, um eine Verbindung 2 mit der folgenden Formel zu erhalten:

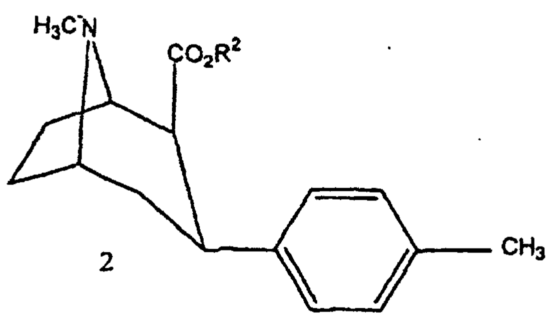

2

2) Umwandlung der Verbindung 2 in die Verbindung 3 mit der Formel:

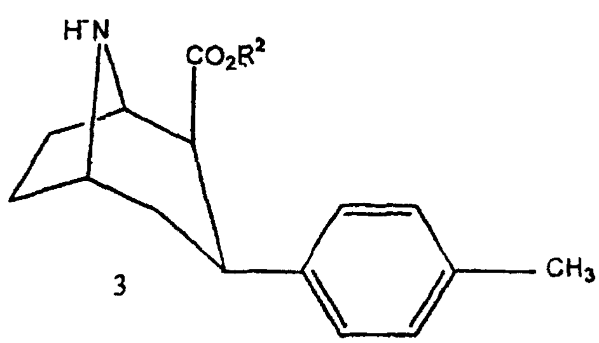

3

durch Umsetzung mit Chlorameisensäuretrichlorethylester und Reduktion mit Zink und Essigsäure,

3) Überführung der Verbindung 3 in die Verbindung 5 mit der Formel:

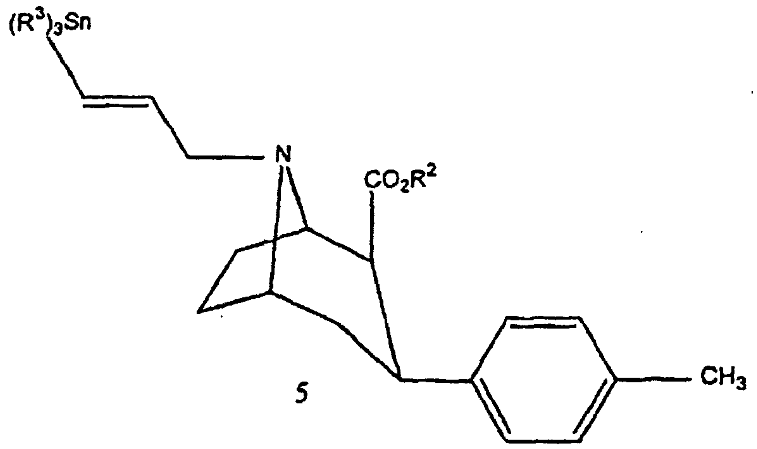

5

in der $R^3$ eine Alkylgruppe ist, und

4) Umsetzung der Verbindung 5 mit dem Iod, um die oben definierte Verbindung der Formel (I) zu erhalten.

**7.** Verfahren zur Herstellung einer Verbindung, die der folgenden Formel entspricht:

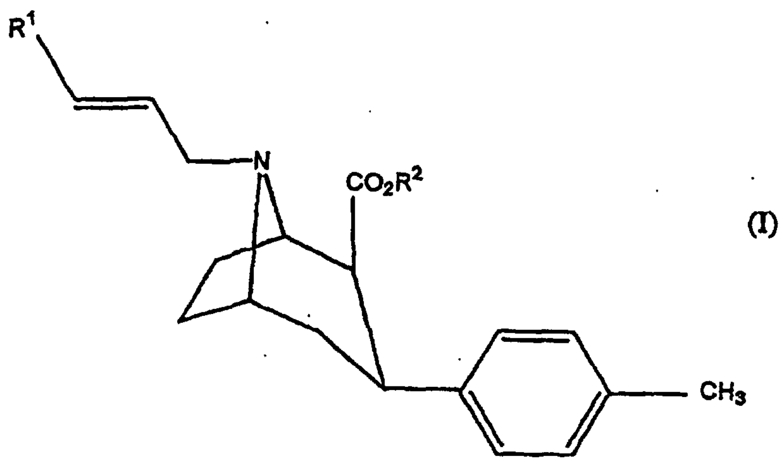

(I)

in der $R^1$ ein radioaktives Isotop von I darstellt und $R^2$ wie in Anspruch 1 definiert ist, **dadurch gekennzeichnet, dass** es die folgenden Stufen umfasst:

1) Umsetzung des Arylmagnesiumbromids der Formel:

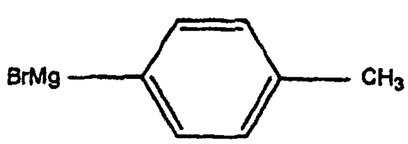

mit einem Ester des Anhydroecgonins 1 der Formel:

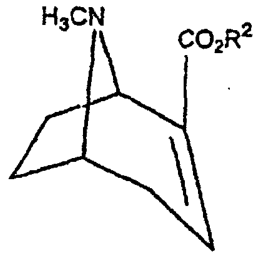

1

in dem $R^2$ die nachstehend gegebene Bedeutung hat, um eine Verbindung 2 mit der folgenden Formel zu erhalten:

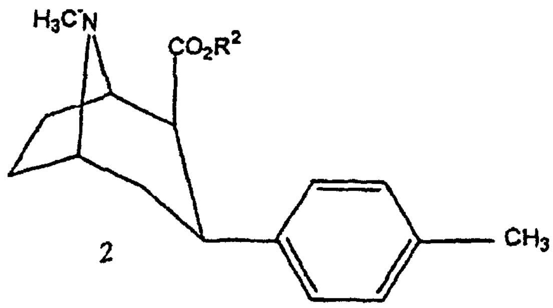

2

2) Umwandlung der Verbindung 2 in die Verbindung 3 mit der Formel:

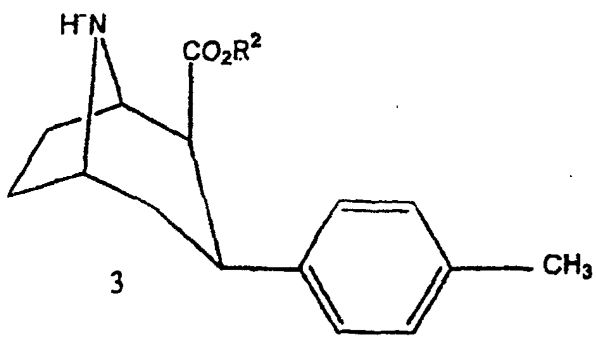

3

durch Umsetzung mit Chlorameisensäuretrichlorethylester und Reduktion mit Zink und Essigsäure,

3) Überführung der Verbindung 3 in die Verbindung 5 mit der Formel:

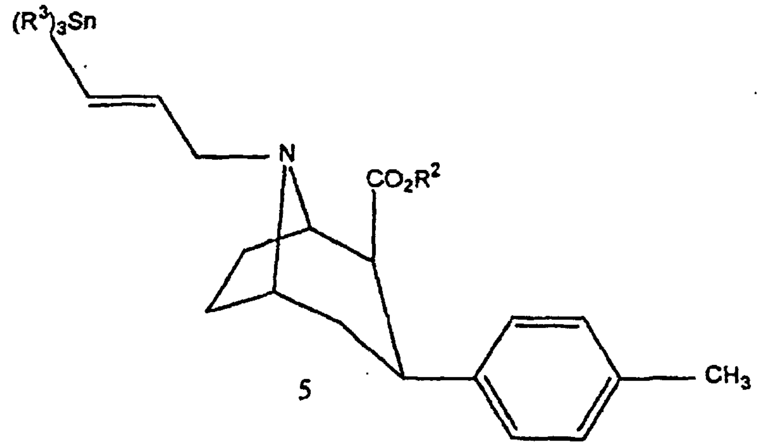

5

in der $R^3$ eine Alkylgruppe ist, und

4) Umsetzung der Verbindung 5 mit dem Iodid der Formel Na*I, in der *I ein radioaktives Isotop des Iods ist, in Gegenwart eines Oxidationsmittels.

**8.** Verfahren nach einem der Ansprüche 6 und 7, **dadurch gekennzeichnet, dass** man in der Stufe 3) die Verbindung 3 direkt in die Verbindung 5 durch Umsetzung mit 3-Chlor-1-trialkylstannylprop-1-en überführt.

**9.** Verfahren nach einem der Ansprüche 6 und 7, **dadurch gekennzeichnet, dass** man in der Stufe 3) zunächst die Verbindung 3 mit 1-Brom-2-propin reagieren lässt, um die Verbindung 4 mit der folgenden Formel zu erhalten:

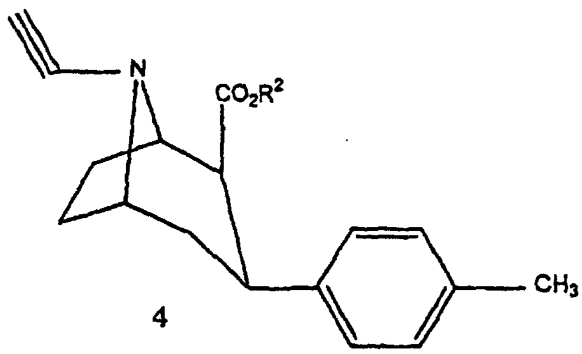

4

und anschließend die Verbindung 4 mit Trialkylstannylhydrid reagieren lässt, um die Verbindung 5 zu erhalten.

**10.** Pharmazeutische Zusammensetzung, **dadurch gekennzeichnet, dass** sie eine Verbindung mit der folgenden Formel:

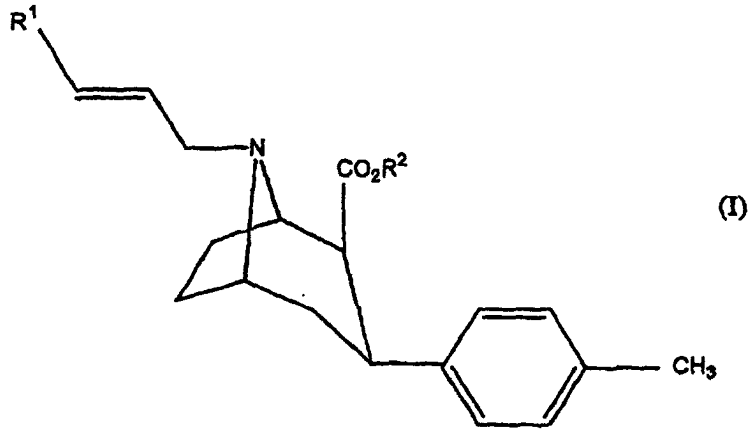

(I)

in der $R^1$ I oder ein radioaktives Isotop von I darstellt und $R^2$ wie in Anspruch 1 definiert ist, und einen pharmazeutisch unbedenklichen Trägerstoff umfasst.

**11.** Radiopharmazeutische Zusammensetzung, **dadurch gekennzeichnet, dass** sie eine Verbindung mit der folgenden Formel:

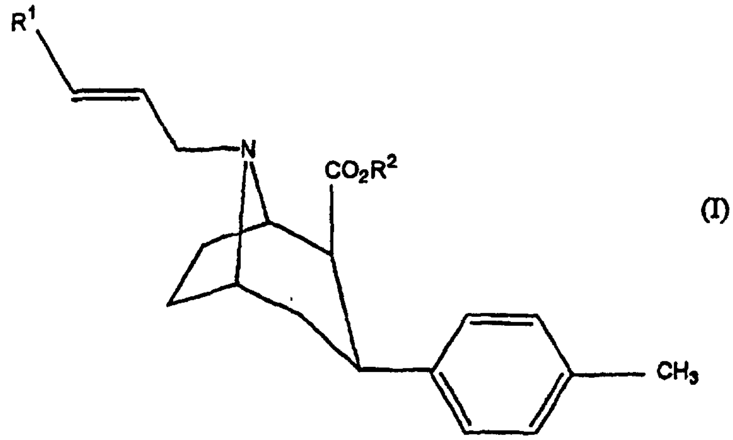

(I)

in der $R^1$ ein radioaktives Isotop von I darstellt, und $R^2$ wie in Anspruch 1 definiert ist, und einen pharmazeutisch unbedenklichen Trägerstoff umfasst.

**12.** Zusammensetzung nach einem der Ansprüche 10 und 11, **dadurch gekennzeichnet, dass** sie in der Form einer Injektionslösung ist.

**13.** Zusammensetzung nach einem der Ansprüche 10 bis 12, **dadurch gekennzeichnet, dass** $R^1$ $^{125}I$, $^{123}I$ oder $^{131}I$ und $R^2$ $CH_3$ darstellt und $R^1$ in der E-Stellung ist.

**14.** Radiopharmazeutische Zusammensetzung nach einem der Ansprüche 11 bis 13 für die Visualisierung von Dopamintransportern im zentralen Nervensystem.

## Claims

**1.** Compound of formula

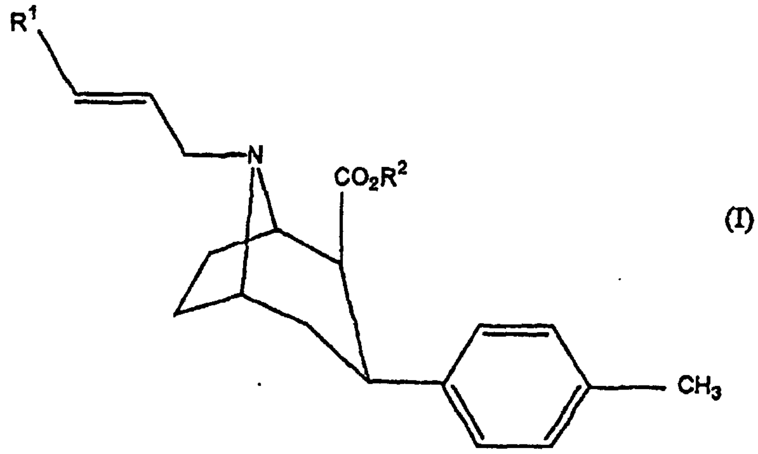

(I)

in which:

- $R^1$ represents I, a radioactive isotope of I or a group of formula $Sn(R^3)_3$ in which $R^3$ is an alkyl group comprising 1 to 8 carbon atoms; and
- $R^2$ represents H; a $C_1$ to $C_6$ alkyl group; a phenyl group; a phenyl group substituted by a halogen atom, a methyl group or a methoxy group; a phenylalkyl or phenylalkenyl group, the alkyl or alkenyl group of which comprises 1 to 6 carbon atoms and the phenyl group of which is optionally substituted by a halogen atom; or a $C_3$ to $C_8$ cycloalkyl group.

2. Compound according to Claim 1, **characterized in that** $R^2$ is a methyl group.

3. Compound according to Claim 1 or 2, **characterized in that** $R^1$ represents $^{125}I$, $^{123}I$ or $^{131}I$.

4. Compound according to Claim 1, **characterized in that** $R^1$ represents $Sn(C_4H_9)_3$, $^{123}I$, $^{125}I$ or $^{131}I$ and $R^2$ represents $CH_3$.

5. Compound according to any one of Claims 1 to 4, **characterized in that** $R^1$ is found in the E position.

6. Process for the preparation of a compound corresponding to the formula:

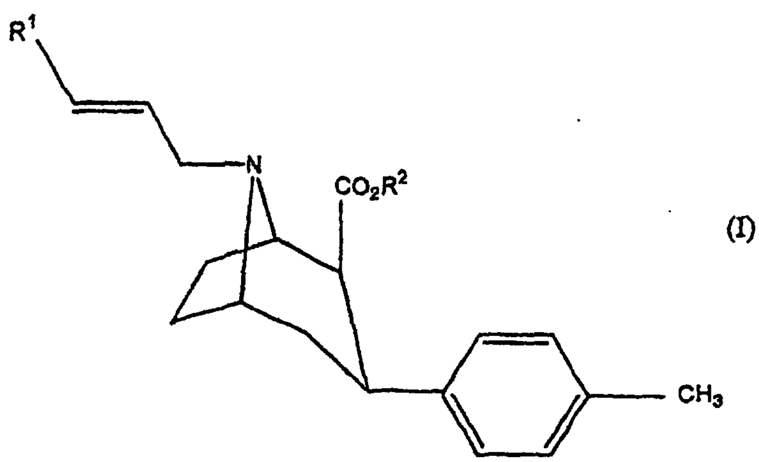

(I)

in which $R^1$ represents I and $R^2$ is as defined in Claim 1, **characterized in that** it comprises the following stages:

1) reaction of the arylmagnesium bromide of formula:

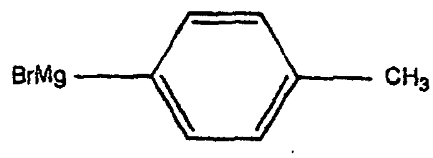

with an ester of anhydroecgonine 1 of formula:

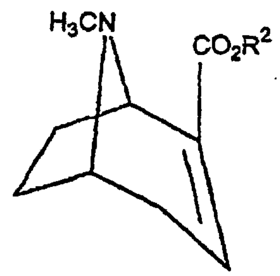

1

in which $R^2$ has the meaning given above, in order to obtain a compound 2 of formula:

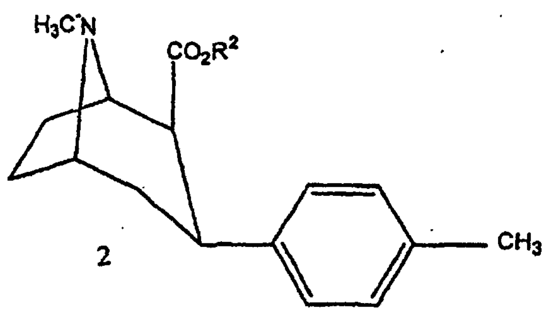

2

2) conversion of the compound 2 to the compound 3 of formula:

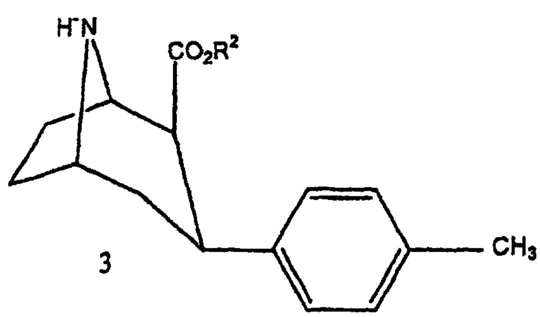

3

by reaction with trichloroethyl chloroformate and reduction with zinc and acetic acid,

3) conversion of the compound 3 to the compound 5 of formula:

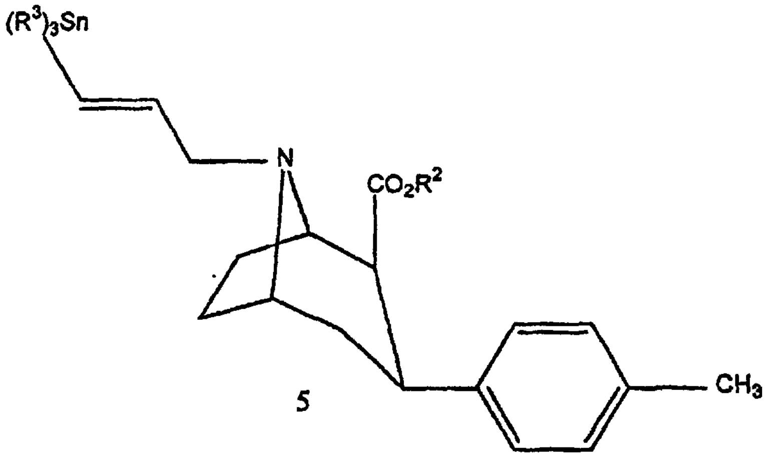

5

in which $R^3$ is an alkyl group, and

4) reaction of the compound 5 with iodine, in order to obtain the compound of formula (I) defined above.

**7.** Process for the preparation of a compound corresponding to the formula:

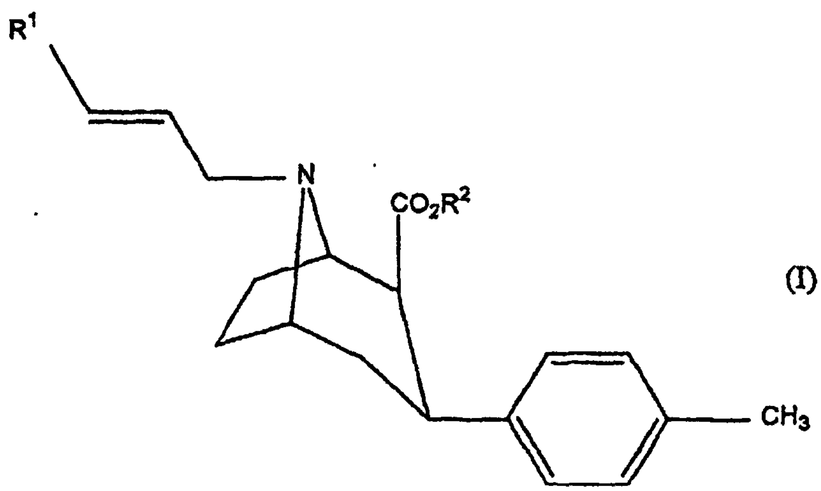

(I)

in which $R^1$ represents a radioactive isotope of I and $R^2$ is as defined in Claim 1, **characterized in that** it comprises the following stages:

1) reaction of the arylmagnesium bromide of formula:

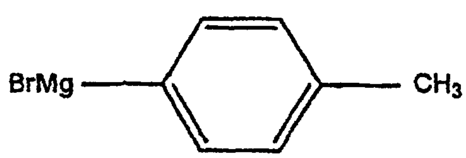

with an ester of anhydroecgonine 1 of formula:

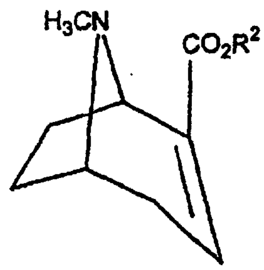

1

in which $R^2$ has the meaning given above, in order to obtain a compound 2 of formula:

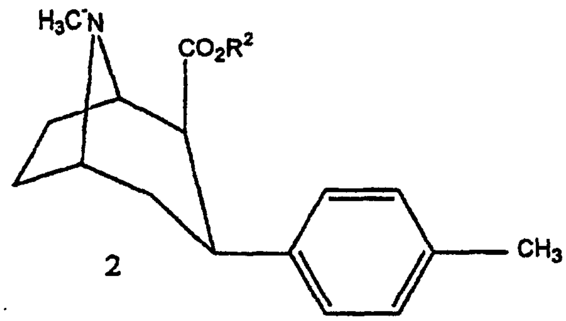

2

2) conversion of the compound 2 to the compound 3 of formula:

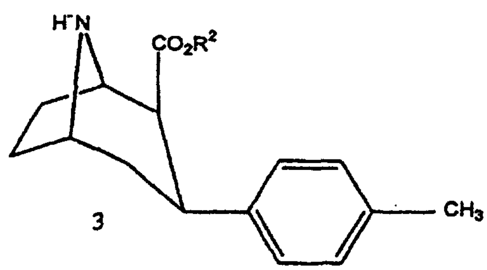

3

by reaction with trichloroethyl chloroformate and reduction with zinc and acetic acid,

3) conversion of the compound 3 to the compound 5 of formula:

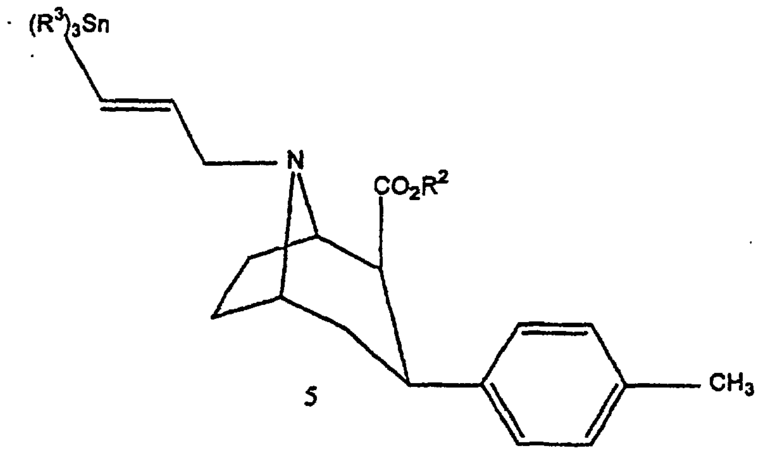

5

in which $R^3$ is an alkyl group, and

4) reaction of the compound 5 with the iodide of formula Na*I, in which *I is a radioactive isotope of iodine, in the presence of an oxidizing agent.

**8.** Process according to either one of Claims 6 and 7, **characterized in that**, in stage 3), the compound 3 is converted directly to the compound 5 by reaction with 3-chloro-1-trialkylstannylprop-1-ene.

**9.** Process according to either one of Claims 6 and 7, **characterized in that**, in stage 3), the compound 3 is first of all reacted with 1-bromo-2-propyne, in order to obtain the compound 4 of formula:

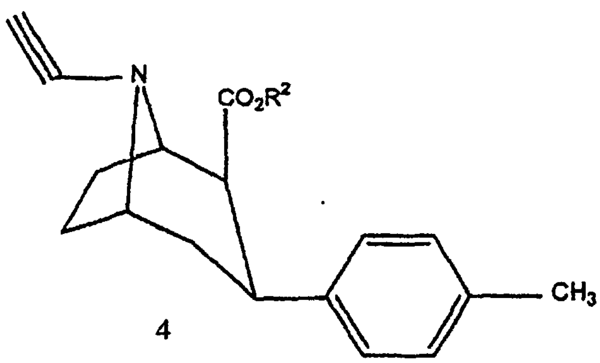

4

and then the compound 4 is reacted with trialkylstannyl hydride, in order to obtain the compound 5.

**10.** Pharmaceutical composition, **characterized in that** it comprises a compound of formula:

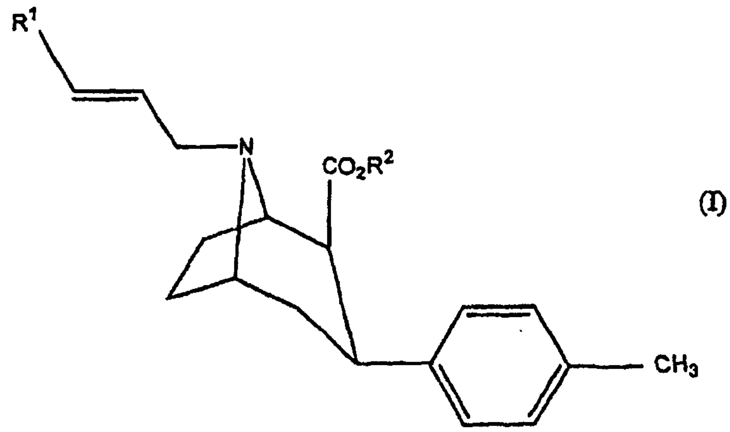

(I)

in which $R^1$ represents I or a radioactive isotope of I; and $R^2$ is as defined in Claim 1, and a pharmaceutically acceptable vehicle.

11. Radiopharmaceutical composition, **characterized in that** it comprises a compound of formula:

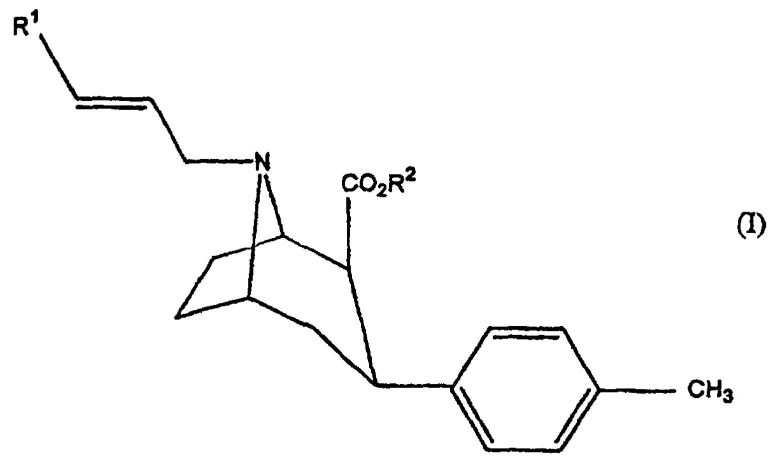

(I)

in which $R^1$ represents a radioactive isotope of I; and $R^2$ is as defined in Claim 1, and a pharmaceutically acceptable vehicle.

12. Composition according to either one of Claims 10 and 11, **characterized in that** it is in the form of an injectable solution.

13. Composition according to any one of Claims 10 to 12, **characterized in that** $R^1$ represents $^{125}I$, $^{123}I$ or $^{131}I$, $R^2$ represents $CH_3$ and $R^1$ is in the E position.

14. Radiopharmaceutical composition according to any one of Claims 11 to 13, for the visualization of dopamine transporters in the central nervous system.

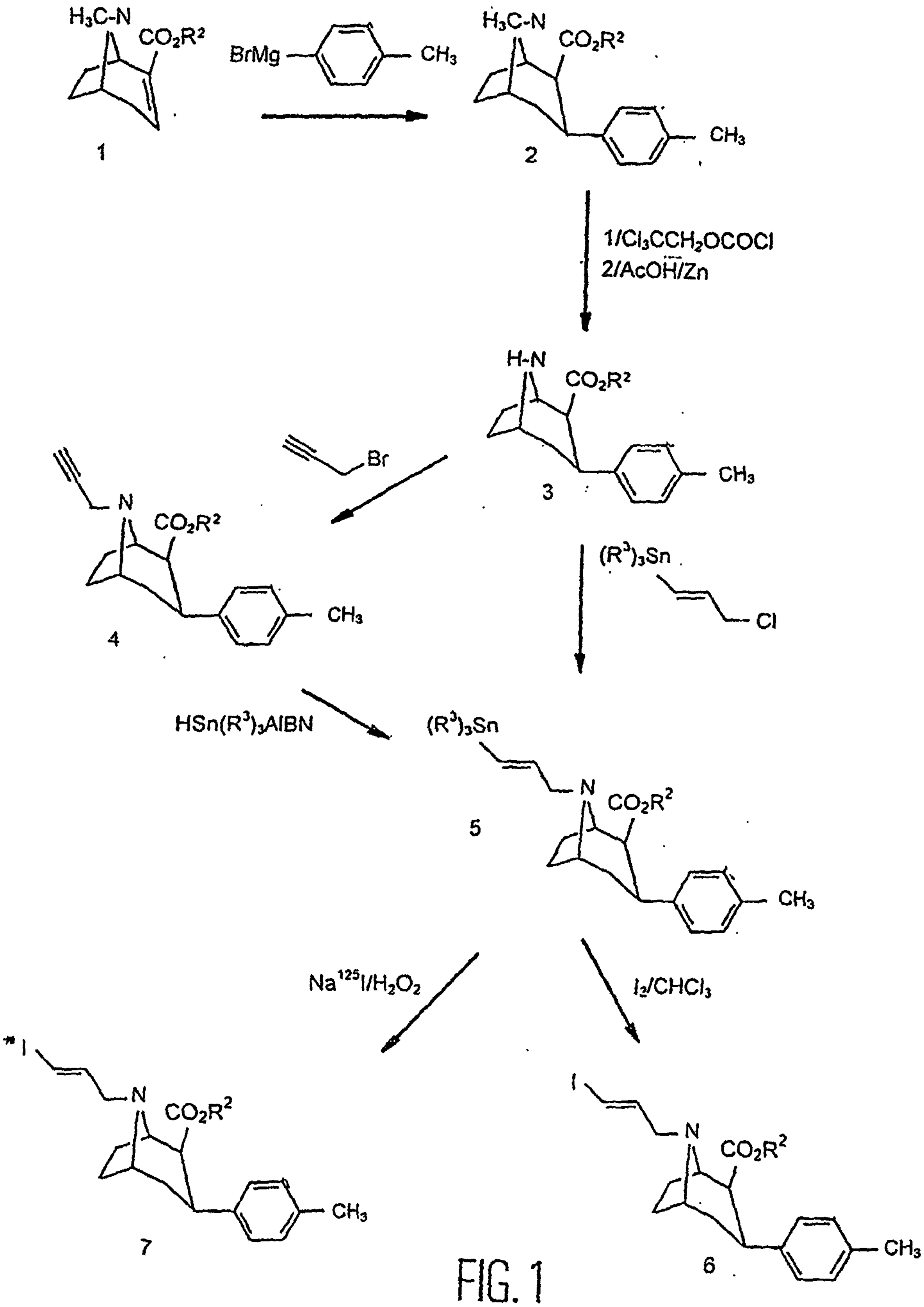
1
2
1/Cl$_3$CCH$_2$OCOCl
2/AcOH/Zn
3
4
HSn(R$^3$)$_3$AIBN
5
Na$^{125}$I/H$_2$O$_2$
I$_2$/CHCl$_3$
7
6

FIG. 1

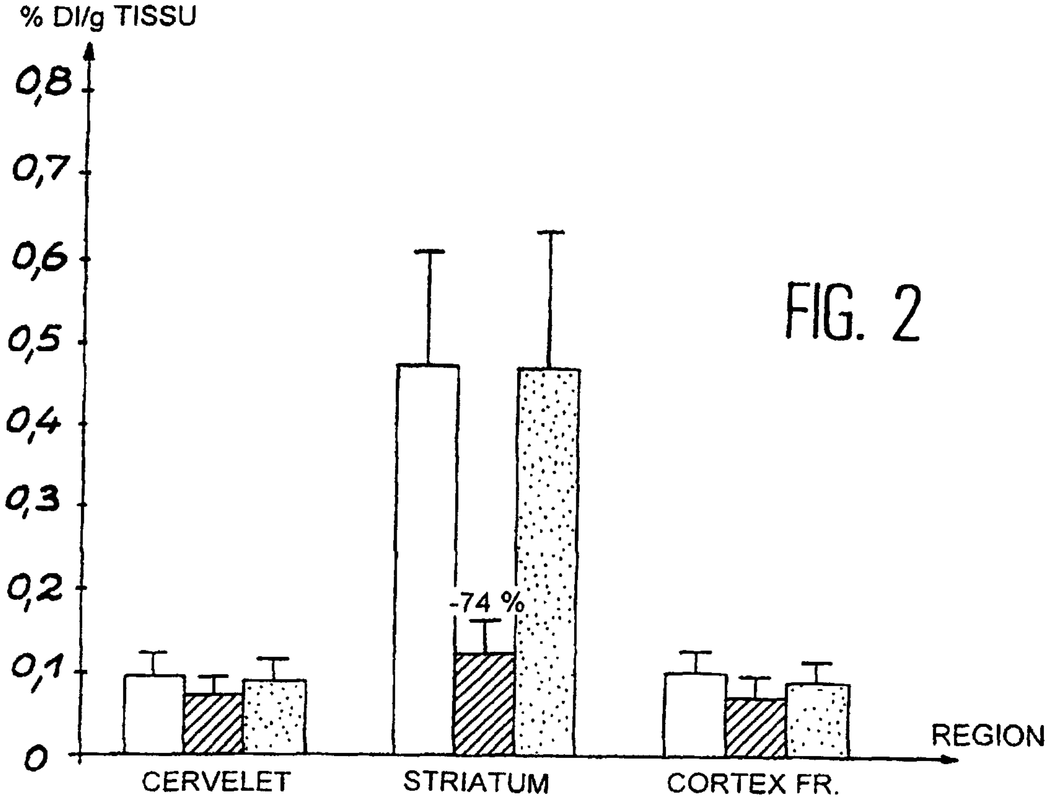
% DI/g TISSU
0,8
0,7
0,6
0,5
0,4
0,3
0,2
0,1
0
-74 %
REGION
CERVELET
STRIATUM
CORTEX FR.

FIG. 2

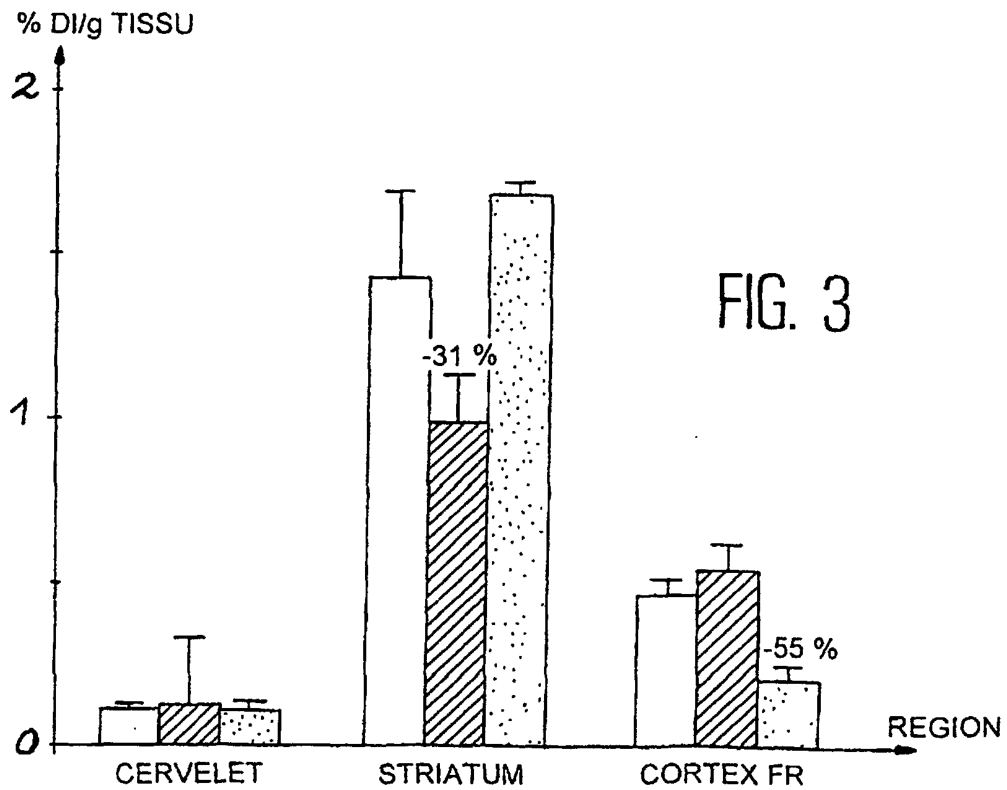
% DI/g TISSU
2
1
0
-31 %
-55 %
REGION
CERVELET
STRIATUM
CORTEX FR

FIG. 3

PRODUIT PE21

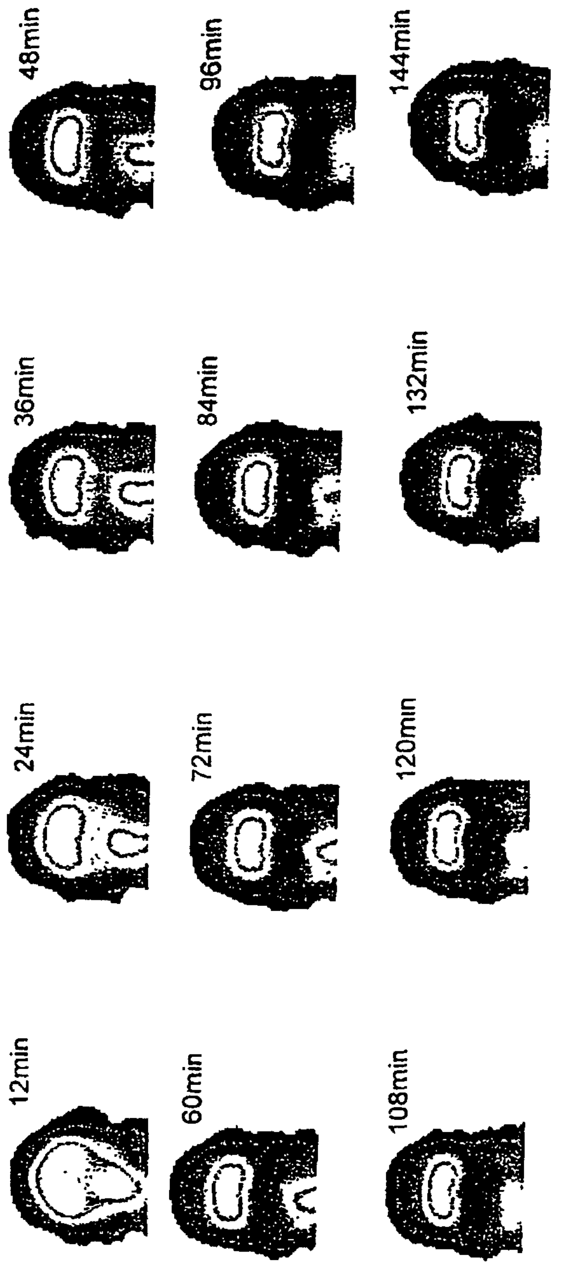


COUPES CORONALES (ADDITION 63 A 73)

FIG. 4